# FASCICULE DE BREVET EUROPEEN

(11) **EP 0 837 749 B1**
(45) Date de publication et mention de la délivrance du brevet: **24.07.2002**
(21) Numéro de dépôt: 96915938.3
(22) Date de dépôt: 14.06.1996
(51) Int. Cl.: B23B 31/107, B25F 3/00, A61B 17/16

(54) **PROCEDE ET DISPOSITIF DE RACCORDEMENT RAPIDE ET INSTRUMENT CHIRURGICAL POUR L'ENTRAINEMENT D'OUTILS ROTATIFS INTERCHANGEABLES**
SCHNELLKUPPLUNGSMETHODE UND VORRICHTUNG UND CHIRUGISCHES INSTRUMENT FÜR DEN ANTRIEB VON AUSWECHSELBAREN ROTIERENDEN WERKZEUGEN
QUICK CONNECTION METHOD AND DEVICE, AND SURGICAL INSTRUMENT FOR DRIVING INTERCHANGEABLE ROTARY TOOLS

(30) Priorité: 14.06.1995 CH 175295
(43) Date de publication de la demande: 29.04.1998
(73) Titulaire: Sodem Diffusion S.A., 1228 Genève (CH)
(72) Inventeur: MENUT, Jean-Baptiste, CH-1219 Châtelaine (CH); PAHUD, Pierre, CH-1018 Lausanne (CH)
(74) Mandataire: Faltas Mikhail, William
(86) Numéro de dépôt international: CH9600228
(87) Numéro de publication internationale: WO9700149

(56) Documents cités:
- CH-A- 575 229
- DE-U- 8 712 362
- DE-U- 8 901 367
- FR-A- 1 424 002

## Description

### Domaine de l'invention

L'invention concerne un dispositif de raccordement rapide d'outils interchangeables à un arbre moteur selon le préambule de la revendication 1 et comme connû du document CH-A-575 229. En particulier elle concerne un instrument chirurgical pour l'entraînement d'outils interchangeables à diverses distances prédéterminées de l'arbre moteur.

### Etat de la technique

Certains instruments chirurgicaux connus, que l'on utilise actuellement pour entraîner divers outils interchangeables à diverses distances d'un arbre moteur, sont munis d'un premier ensemble d'outils spéciaux et d'un second ensemble de broches de guidage, dont la longueur varie selon la distance requise de cas en cas entre le tête de coupe et l'arbre moteur.

Dans ces instruments connus, il est prévu que chaque outil spécial soit d'abord raccordé à l'arbre moteur au moyen d'une pince de serrage et que chaque broche de guidage soit ensuite vissée sur un boîtier entourant l'arbre moteur. Ce serrage et ce vissage sont par ailleurs réalisés à l'aide de deux clés spéciales pour assurer une liaison adéquate des outils. à l'arbre moteur, et entraînent ainsi une perte du temps requis pour le remplacement des outils.

De plus, lesdits instruments doivent être munis d'un nombre considérable d'outils spéciaux, ce nombre étant égal au nombre de types de têtes de coupe requises, multiplié par le nombre de longueurs requises pour assurer les différentes distances entre les têtes de coupe et l'arbre moteur. Or, cela peut entraîner des frais d'outillage considérables ainsi que des problèmes de stockage, étant donné qu'il convient d'assurer toujours un stock adéquat de ces outils spéciaux qui sont relativement coûteux de types et de longueurs différentes et qui s'usent plus ou moins rapidement selon l'usage qu'on fait des divers outils.

L'état de la technique relative aux dispositifs de raccordement d'outils comprenant des moyens d'autoserrage et de blocage peut être illustré par les brevets suivants.

CH 575 229 a pour objet une perceuse chirurgicale comprenant un arbre moteur dans une première enveloppe, une tête amovible pourvue d'un arbre entraîné dans une deuxième enveloppe, d'un outil de coupe pouvant être accouplé audit arbre entraîné, un organe d'embrayage fixé à l'arbre moteur et une mordache fixée audit arbre entraîné qui viennent en position d'entraînement lorsque lesdites enveloppe sont reliées.

CH 573 743 a pour objet une perceuse chirurgicale comprenant un arbre moteur dans un boîtier, une arbre entraîné intermédiaire couplé à l'arbre moteur monté dans un boîtier arrière couplé avec le boîtier de l'arbre moteur et relié par un engrenage à un arbre entraîné avant associé à un dispositif de fixation de l'outil et monté dans un boîtier avant muni d'un embout qui est amovible pour permettre le serrage de ce dispositif de fixation.

CH 671 174 a pour objet un mandrin autoserrant destiné à éliminer les inconvénients de la nécessité d'utiliser une clé de serrage dans les instruments portatifs à usage chirurgical couramment utilisés.

CH 512 954 a pour objet un dispositif de blocage d'outils sur un mandrin et CH 664 516 a pour objet un dispositif de blocage d'un outil dans un rotor.

Les dispositifs de raccordement d'outils et les instruments connus mentionnés ci-dessus ne répondent cependant pas d'une manière vraiment satisfaisante à toutes les exigences les plus strictes quant à la rapidité, la simplicité constructive et la fiabilité des moyens techniques utilisés, sans l'aide d'une clé ou tout autre outil accessoire..

### But de l'invention

L'invention a pour but de fournir un dispositif de raccordement rapide d'outils interchangeables et notamment un instrument chirurgical comprenant un tel dispositif de raccordement, permettant d'obvier aux inconvénients mentionnés des dispositifs de raccordement et instruments connus et présentant une construction aussi simple, compacte et fiable que possible de manière à pouvoir assurer le raccordement et le verrouillage d'une pluralité d'outils de coupe rotatifs interchangeables, dans un minimum de temps et sans nécessiter l'aide du moindre moyen auxiliaire, et cela plus particulièrement de manière à pouvoir actionner les outils de coupe à des distances prédéterminées différentes de l'arbre d'entraînement.

### Résumé de l'invention

La présente invention telle que définie dans les revendications a pour objet un procédé et un dispositif de raccordement rapide d'outils interchangeables et un instrument chirurgical muni d'un tel dispositif de raccordement et destiné notamment à l'entraînement d'outils interchangeables ä différentes distances d'un arbre moteur.

La présente invention consiste essentiellement à assurer le raccordement rapide de divers outils rotatifs interchangeables à un moteur au moyen d'une combinaison de moyens techniques simples et fiables comprenant:
(a) des outils standard comportant des tiges identiques dont le diamètre est adapté à un tube porte-outil rotatif monté dans un support tubulaire,
(b) un premier raccord rapide qui assure la fixation de l'outil dans le tube porte-outil,
(c) un second raccord rapide permettant le montage du support tubulaire sur le boîtier de l'arbre moteur,
(d) des moyens de couplage de la tige de l'outil à l'arbre moteur et
(e) des moyens de verrouillage permettant de bloquer et de libérer le premier raccord rapide lorsque le second raccord rapide est respectivement engagé ou désengagé.

L'invention peut être illustrée à l'aide des formes d'exécution d'un dispositif de raccordement rapide et d'un instrument chirurgical décrites ci-après à titre d'exemple et représentées dans le dessin. Les éléments analogues portent les mêmes référence dans les figures du dessin, dans lequel:
Fig. 1 est une vue en coupe longitudinale d'une forme d'exécution d'un dispositif de raccordement rapide selon l'invention associé à un outil et un arbre moteur.
Fig. 2 est une vue en coupe longitudinale d'une forme d'exécution d'un instrument chirurgical comprenant un dispositif de raccordement rapide selon l'invention associé à un outil et à un arbre moteur.

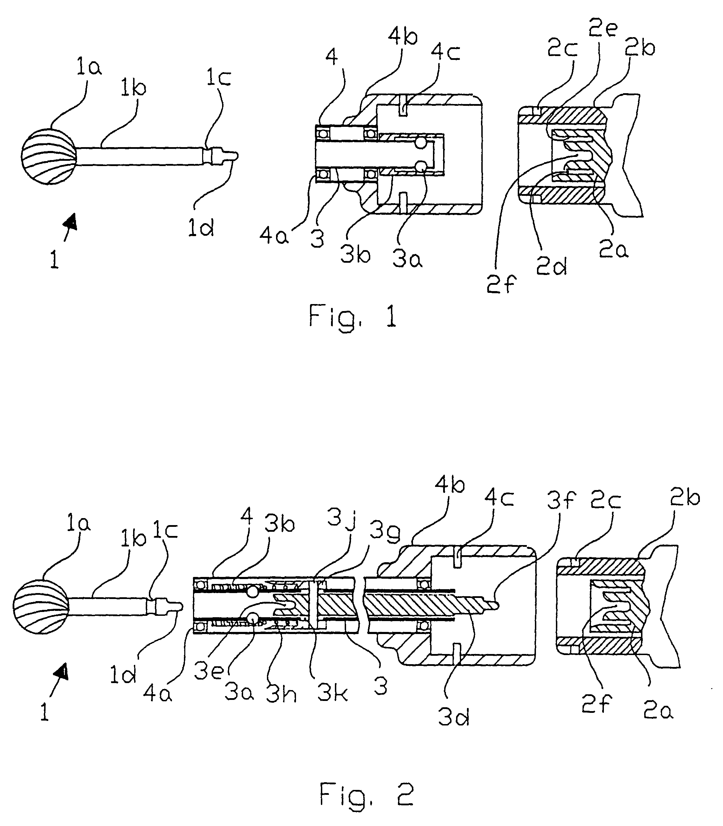

Fig. 1 montre un dispositif de raccordement rapide selon l'invention destiné à l'entraînement d'un outil de coupe interchangeable 1 par un arbre moteur 2a qui est muni d'une fente de couplage 2f et est monté dans un boîtier 2b muni d'une rainure de verrouillage 2c.

L'outil de coupe 1 associé à ce dispositif de raccordement comprend une tête de coupe 1a solidaire d'une tige ronde 1b qui présente un diamètre et une longueur prédéterminés et est munie d'une rainure annulaire 1c et d'un ergot de couplage 1d.

La forme et les dimensions de l'outil de coupe 1, de l'arbre moteur 2a et de son boîtier 2b sont adaptées au dispositif de raccordement

Le dispositif de raccordement rapide selon l'invention comprend essentiellement un tube porte-outil rotatif 3 monté coaxialement dans un support tubulaire 4 et agencé dans des paliers 4a de manière à tourner librement autour de l'axe commun de ces tubes 3 et 4.

Le diamètre du tube porte-outil 3 est choisi de manière qu'il soit légèrement supérieur au diamètre de la tige 1b et qu'il permette le guidage et le montage de l'outil de coupe 1 dans ce tube 3 avec un faible jeu. La distance requise entre la tète de coupe 1a et l'arbre moteur 2a est déterminée par la longueur de ce tube 3. Cette longueur est adaptée en l'occurrence à la longueur de la tige 1b de l'outil de manière que l'ergot de couplage 1d de cette tige 1b fasse saillie ä l'extrémité arrière du tube 3 lorsque l'outil est monté dans ce tube.

Un premier raccord rapide servant au montage de l'outil dans le tube porte-outil 3 comprend en l'occurrence deux billes mobiles radialement 3a qui sont logées dans deux trous diamétralement opposés ménagés dans ce tube 3 et sont maintenues en place dans ces trous par une douille élastique fendue 3b montée fixe sur le tube porte-outil 3.

Ainsi, comme il ressort de la fig. 1. les billes 3a pénètrent normalement à l'intérieur du tube porte-outil 3 sous l'action de la douille 3b, tandis qu'elles sont engagées dans la rainure 1c de la tige 1b lorsque l'outil est monté dans le tube porte-outil 3. Son ergot de couplage 1d fait alors saillie à l'extrémité arrière de ce tube porte-outil 3 afin de permettre l'engagement de cet ergot 1d dans la fente le couplage 2f de l'arbre moteur 2a.

L'arbre moteur 2a est muni d'une cavité annulaire 2d délimitée par une paroi extérieure annulaire 2e et dimensionnée de manière à pouvoir y loger la partie arrière de la douille élastique 3b.

La fente de couplage 2f est adaptée en l'occurrence à l'ergot de couplage 1d de manière que l'outil de coupe soit couplé directement à l'arbre 2a lorsque l'ergot 1d est engagé dans la fente 2f de cet arbre.

Un second raccord rapide associé au support tubulaire 4 et au boîtier 2b de l'arbre moteur 2a est constitué par un raccord à emboîtement du type à baïonnette comprenant une douille 4b qui est solidaire de ce tube 4 à son extrémité arrière et est munie de deux goujons intérieurs de verrouillage diamétralement opposés 4c. Cette douille 4b forme la partie femelle dudit second raccord rapide du type à baïonnette. Le boîtier 2b de l'arbre moteur 2a présente deux rainures de verrouillage 2c et il est adapté à la douille 4b de manière qu'il constitue la partie mâle du second raccord du type à baïonnette.

Le dispositif de raccordement décrit et représenté en fig. 1 permet d'assurer le montage et le démontage des outils interchangeables en deux étapes très simples et rapides.

L'outil de coupe requis est d'abord raccordé au tube porte-outil 3 dans une première étape très brève qui consiste à enfoncer simplement la tige 1b à l'extrémité avant de ce tube porte-outil 3 jusqu'à ce que les billes 3a mobiles s'engagent dans la rainure 1c de la tige de l'outil et que l'ergot 1d de cette tige soit disposée en saillie de l'extrémité arrière du tube porte-outil 3.

Or, le déplacement axial de la tige 1b repousse les billes vers l'extérieur de ce tube 3, tandis qu'elles sont ensuite repoussées radialement vers l'intérieur par la douille 3b et s'engagent enfin dans la rainure 1c de la tige 1b, permettant d'empêcher ainsi tout déplacement axial de la tige 1b. L'ergot 1d de la tige 1b de l'outil faisant saillie à l'extrémité arrière du tube porte-outil 3 permet alors d'accoupler l'outil 1 en même temps que le tube porte-outil 3 à l'arbre moteur 2a.

Le support tubulaire 4 est ensuite raccordé au boîtier 2b de l'arbre moteur 2a dans une seconde étape très brève qui consiste simplement à enfiler le boîtier 2b de l'arbre moteur 2a dans la douille à baïonnette 4b du support tubulaire 4 de manière que les goujons 4c soient déplacés vers l'arrière dans les rainures de verrouillage 2d et assurent le verrouillage en y effectuant une rotation relative. Or, ce second raccordement assure en même temps le couplage de l'outil 1 et du tube porte-outil rotatif 3 à l'arbre 2a grâce à l'engagement de l'ergot 1d de l'outil dans la fente 2f de l'arbre 2a.

L'extrémité arrière de la douille élastique 3b est ainsi engagée dans la cavité annulaire 2d de l'arbre 2a et la paroi annulaire 2e vient entourer cette douille élastique 3b, assurant ainsi le blocage des billes 3a dans la rainure 1c de l'outil et par conséquent le verrouillage de l'outil dans le tube 3.

Fig. 2 montre une forme d'exécution d'un instrument chirurgical muni d'un dispositif de raccordement rapide selon l'invention comprenant une broche intermédiaire dont la structure est conçue spécialement pour permettre le raccordement rapide et fiable d'outils interchangeables 1 à plusieurs distances prédéterminées de l'arbre moteur associé à cet instrument.

L'instrument chirurgical muni du dispositif de raccordement représenté en fig. 2 permet de combiner avantageusement un nombre quelconque d'outils interchangeables, ayant des tiges identiques et les diverses têtes de coupe requises, avec un nombre quelconque de broches intermédiaires interchangeables ayant la même structure et les longueurs différentes requises pour modifier la distance entre l'outil et l'arbre moteur de cas en cas.

Fig. 2 représente un seul outil de coupe et une seule broche intermédiaire, dont la structure décrite ci-après est essentiellement la même pour l'ensemble des outils et des broches intermédiaires pouvant être utilisées dans diverses combinaisons d'outils et de broches dans le cadre de l'invention.

L'instrument chirurgical représenté en fig. 2 sert à entraîner un outil de coupe 1 par l'intermédiaire d'un dispositif de raccordement rapide comprenant une broche intermédiaire composée d'un tube porte-outil rotatif 3 monté dans un tube support 4 au moyen de paliers 4a. Le tube porte-outil rotatif 3 est muni de billes 3a et d'une douille élastique 3b agencées de la manière déjà décrite pour la fig. 1.

Une pièce de couplage mobile 3d est montée dans ce tube porte-outil 3, présente une fente de couplage intermédiaire 3e destinée à recevoir l'ergot de couplage 1d de l'outil lorsque ce dernier est monté dans le tube 3 et est munie en outre d'un ergot de couplage 3f qui fait saillie de l'extrémité arrière du tube porte-outil 3 et est adapté à s'engager dans la fente de couplage 2f de l'arbre moteur 2a.

Comme il ressort en outre de la fig. 2, une douille de verrouillage mobile 3g est agencée sur le tube porte-outil 3, est séparée de la douille élastique 3b par un ressort de pression 3h et est fixée sur la pièce de couplage mobile 3d au moyen d'une goupille 3j traversant deux fentes longitudinales opposées 3k ménagées dans le tube 3.

Ladite broche intermédiaire est en outre associée à un second raccord rapide à emboîtement du type à baïonnette comprenant les éléments suivants déjà décrits par rapport à la fig. 1: la douille 4b avec les goujons 4c, constituant l'élément femelle de ce second raccord rapide, et le boîtier 2b avec la rainure de verrouillage 2c, constituant l'élément mâle du second raccord rapide.

Quant au fonctionnement du raccord décrit, l'outil 1 est d'abord monté à l'extrémité avant du tube 3 en y introduisant la tige 1b de manière qu'elle écarte les billes 3a contre l'action de la douille élastique 3b afin de permettre le déplacement axial de cette tige 1b jusqu'à ce que l'ergot de couplage 1d soit engagé dans la fente de couplage 3e de la pièce de couplage intermédiaire 3d, les billes étant alors repoussées vers l'intérieur par la douille fendue et engagées dans la rainure 1c et l'outil étant ainsi positionné correctement et couplé au tube porte-outil 3 au moyen de la pièce de couplage 3d.

Le tube support 4 est ensuite raccordé au boîtier 2b de l'arbre 2a par l'intermédiaire dudit raccord à baïonnette, le tube porte-outil 3 étant couplé en même temps à l'arbre moteur 2a. Ainsi, lorsque le boîtier 2b est inséré dans la douille de raccordement 4b du tube support 4, l'ergot 3f de la pièce de couplage intermédiaire 3d s'engage dans la fente 2f de l'arbre d'entraînement 2a et vient buter contre le fond de cette fente 2f.

Par conséquent, le raccordement de la broche intermédiaire au boîtier 2b entourant l'arbre moteur 2a provoque un déplacement de la pièce de couplage intermédiaire 3d et provoque ainsi le déplacement de la douille de verrouillage 3g contre l'action du ressort de pression 3h de manière que cette douille 3g vienne entourer la douille élastique 3b. On empêche ainsi toute expansion de cette douille fendue 3b, assurant ainsi le blocage des billes 3a dans 1a rainure 1c de l'outil.

On obtient ainsi un raccordement solide de tous les éléments rotatifs d'une part (outil 1, tube porte-outil 3, pièce 3d, arbre moteur 2a) et fixes d'autre part (tube support 4, boîtier 2b).

L'instrument décrit est muni avantageusement conformément à l'invention d'une pluralité de fraises de types différents de la même longueur, d'une pluralité de broches intermédiaires ayant des longueurs différentes et des moyens de fixation rapide et de verrouillage permettant de monter lesdites fraises sélectivement à des distances prédéterminées de l'arbre moteur par l'intermédiaire desdites broches.

Ainsi, on nécessite pour cet instrument uniquement un jeu de fraises de même longueur et de type différents que l'on peut monter sélectivement sur lesdites broches intermédiaires de longueur différentes afin d'effectuer l'ensemble des interventions chirurgicales requises de cas en cas.

Grâce à l'emploi d'un jeu desdites broches intermédiaires, il devient possible de fixer n'importe quelle fraise sur n'importe quelle broche intermédiaire et de réduire le nombre de fraises de même longueur au nombre de types de tête de fraise requises

La fixation rapide et le verrouillage de chaque fraise sont assurés par des moyens techniques particulièrement simples et fiables qui sont prévus spécialement conformément à l'invention de manière que les fonctions de fixation et de verrouillage soient découplées

Ainsi, dans les formes d'exécution décrites, le montage de chaque fraise sur une broche de longueur prédéterminée est assuré d'abord par un encliquetage à ressort, tandis que le verrouillage de l'encliquetage est actionné lorsque la broche est ensuite montée sur le boîtier de l'arbre moteur.

Dans les formes d'exécution décrites de l'invention, l'encliquetage de la fraise et son verrouillage n'assurent que la fixation longitudinale de la fraise, tandis que la rotation de la fraise est assurée par des ergots et des fentes associés à la tige de la fraise et à l'arbre moteur.

Il est cependant possible d'envisager que la fonction de verrouillage de l'encliquetage assure simultanément la fonction d'entraînement de la fraise, par exemple par une pince actionnée par le mécanisme de verrouillage.

L'essentiel est que l'encliquetage de la fraise se fasse à la main lorsque le tube-support de la broche n'est pas monté sur le boîtier de l'arbre moteur, que l'opérateur sente bien lorsque la fraise est mise correctement en place, et que le verrouillage de l'encliquetage se fasse lors du montage de la broche sur ledit boîtier de l'arbre moteur.

### Avantages

La combinaison spéciale des caractéristiques prévues conformément à l'invention permet de réaliser une combinaison particulière de divers avantages pratiques que l'on peut expliquer de la manière suivante.

Le raccordement ainsi que le remplacement des outils s'obtient d'une façon sûre en un minimum de temps, sans aucun outil accessoire.

On peut assurer une grande variété de combinaisons de types de fraises et de longueurs de l'instrument, tout en limitant le nombre de fraises requises au strict minimum. Ainsi par exemple, un moteur muni de 6 types de fraise de même longueur et de 3 broches intermédiaires de longueurs différentes permet le choix de 18 combinaisons du types de fraise et de longueur de l'instrument.

Cela permet d'une part une économie importante des frais d'outillage par rapport aux instruments connus, qui nécessiteraient, dans ce cas particulier donné à titre d'exemple, 18 fraises de six types différents et de 3 longueurs différentes, ainsi que 3 broches de longueurs différentes.

### Applications de l'invention

L'invention se prête au raccordement rapide d'outils interchangeables destinés à diverses utilisations et s'applique plus particulièrement aux instruments chirurgicaux qui sont destinés à la chirurgie orthopédique et doivent permettre notamment le fonctionnement d'outils de coupe à diverses distances d'un arbre moteur.

## Revendications

1. Dispositif de raccordement rapide d'outils rotatifs interchangeables à un arbre moteur disposé dans un boîtier raccordé à un support amovible associé à des moyens de couplage d'un outil à l'arbre moteur, **caractérisé en ce que** le dispositif comprend:
(a) un tube porte-outil rotatif (3) monté coaxialement dans un support tubulaire (4),
(b) un premier raccord rapide comprenant des moyens de fixation (3a, 3b) d'un outil (1) dans ledit tube porte-outil (3),
(c) un second raccord rapide (4b, 4c, 2b, 2c) associé audit support tubulaire (4) et audit boîtier (2b) disposé autour de l'arbre moteur (2a) et
(d) des moyens de verrouillage (3b, 2d, 2e) associés audit tube porte-outil (3) et à l'arbre moteur (2a) de manière à provoquer le blocage dudit premier raccord rapide au moyen dudit second raccord rapide.

2. Dispositif de raccordement rapide selon la revendication 1, **caractérisé en ce que** ledit premier raccord rapide comprend un dispositif d'encliquetage (3a, 3b, 1c) permettant l'insertion et la fixation d'un outil dans le tube porte-outil (3) ainsi que la libération rapide de l'outil.

3. Dispositif selon la revendication 2, **caractérisé en ce que** ledit premier raccord comprend des billes mobiles (3a) logées dans des trous ménagés dans ledit tube porte-outil (3) et associées à une douille élastique (3b) fixée sur ce tube porte-outil (3) de manière que ces billes soient maintenues dans une position intérieure dans laquelle elles pénètrent dans le tube porte-outil (3) afin de permettre la fixation de l'outil et que ces billes puissent être repoussées vers l'extérieur afin de libérer l'outil.

4. Dispositif selon la revendication 2 ou 3, **caractérisé en ce que** le diamètre dudit tube porte-outil (3) est adapté à des outils interchangeables comprenant une tige ronde présentant un diamètre permettant son montage avec un faible jeu dans le tube porte-outil (3), une rainure annulaire (1c) associée audit dispositif d'encliquetage et un ergot de couplage (1d).

5. Dispositif selon l'une des revendications 1 à 4, **caractérisé en ce que** ledit second raccord rapide est prévu sous forme d'un raccord à emboîtement, les extrémités à raccorder dudit boîtier (2b) de l'arbre moteur (2a) et du support tubulaire (4) étant agencées de manière qu'elles constituent respectivement les parties mâle et femelle dudit raccord à emboîtement.

6. Dispositif selon la revendication 5, **caractérisé en ce que** l'extrémité dudit support tubulaire (4) à raccorder audit boîtier (2b) de l'arbre moteur (2a) est munie d'une douille (4b) constituant la partie femelle et ce boîtier (2b) est agencé de manière qu'il constitue la partie mâle dudit raccord à emboîtement.

7. Instrument chirurgical muni d'un dispositif de raccordement rapide selon l'une des revendications 1 à 6 et destiné à l'entraînement d'outils de coupe interchangeables à différentes distances prédéterminées d'un arbre moteur, **caractérisé en ce que**:
(a) l'instrument est muni d'un jeu de broches intermédiaires interchangeables associées à un jeu d'outils interchangeables (1) munies de tiges identiques, ces broches présentant chacune une longueur qui correspond à une distance prédéterminée entre chaque outil et l'arbre moteur (2), chacune de ces broches étant composée d'un tube porte-outil rotatif (3) monté dans un tube support (4) et lesdits outils interchangeables (1) présentant des têtes de coupe (1a) de formes différentes et des tiges identiques (1b) ayant un diamètre et une longueur prédéterminés,
(b) ledit tube porte-outil (3) présente un diamètre intérieur permettant l'insertion de la tige (1a) de chaque outil (1) avec un faible jeu, un premier raccord rapide étant associé à ce tube porte-outil (3) de manière à permettre la fixation de chaque outil et des moyens de couplage étant prévus de manière à permettre le couplage de l'outil à l'arbre moteur (2a) par l'intermédiaire de ce tube porte-outil (3),
(c) ledit tube support (4) est associé à un second raccord rapide agencé de manière à permettre la fixation de ce tube support (4) au boîtier (2b) de l'arbre moteur (2a) et
(d) lesdits premier et second raccords rapides sont associés à des moyens de verrouillage dont l'action est conjuguée de telle manière que le blocage du premier raccord rapide est assuré au moyen du second raccord rapide, afin d'assurer ainsi la fixation de l'outil, tandis que la séparation du second raccord rapide permet la libération de l'outil.

8. Instrument chirurgical selon la revendication 7, **caractérisé par le fait que** ledit premier raccord rapide comprend un dispositif d'encliquetage (3a, 3b) permettant l'insertion et la fixation d'un outil dans le tube porte-outil (3) ainsi que la libération rapide de l'outil.

9. Instrument chirurgical selon la revendication 8, **caractérisé en ce que** ledit premier raccord comprend des billes mobiles (3a) logées dans des trous ménagés dans ledit tube porte-outil (3) et associées à une douille élastique (3b) fixée sur ledit tube porte-outil (3) et agencée de manière que ces billes soient maintenues dans une position intérieure dans laquelle elles pénètrent dans le tube porte-outil (3) afin de permettre la fixation de l'outil et que ces billes puissent être repoussées vers l'extérieur afin de libérer l'outil.

10. Instrument chirurgical selon l'une des revendications 8 ou 9, **caractérisé en ce qu'**il est muni d'un jeu d'outils interchangeables comprenant des tiges rondes identiques (1b) qui présentent un diamètre permettant son montage avec un faible jeu dans le tube porte-outil (3), une rainure annulaire (1c) associée audit dispositif d'encliquetage et un ergot de couplage (1d).

11. Procédé de raccordement rapide d'outils rotatifs interchangeables au moyen du dispositif selon la revendication 1, **caractérisé en ce que**:
(a) l'on utilise des outils standard comportant des têtes de coupe de toute forme appropriée fixées sur des tiges rondes identiques qui présentent une longueur et un diamètre prédéterminés, ce diamètre étant adapté audit tube porte-outil rotatif de manière que la tige de chaque outil puisse coulisser avec un faible jeu dans ce tube porte-outil,
(b) l'on adapte la longueur dudit porte-outil à la distance requise entre ledit arbre moteur et la tête de coupe de chaque outil monté dans ce tube porte-outil, ce tube porte-outil rotatif étant monté dans ledit support tubulaire de manière qu'il puisse tourner dans ce support,
(c) l'on raccorde chacun desdits outils audit tube porte-outil au moyen dudit premier raccord rapide de manière à fixer sa position axiale dans ledit tube-porte-outil, et
(d) l'on raccorde ledit support tubulaire audit boîtier de l'arbre moteur au moyen dudit second raccord rapide de manière à effectuer le couplage dudit arbre moteur avec la tige de l'outil monté dans ledit tube porte-outil et à effectuer en même temps le verrouillage dudit second raccord et dudit premier raccord, le tout de manière à rendre chaque outil ainsi raccordé complètement solidaire du tube porte-outil rotatif et de l'arbre moteur et à pouvoir en outre libérer cet outil aussitôt que ledit second raccord rapide est séparé.

## Patentansprüche

1. Einrichtung zur Schnellkupplung von auswechselbaren drehenden Werkzeugen mit einer Antriebswelle in einem Gehäuse, das mit einer abnehmbaren Vorrichtung mit Mitteln zur Kupplung eines Werkzeugs mit der Antriebswelle verbunden ist, **dadurch gekennzeichnet, dass** sie folgendes umfasst:
(a) ein drehendes Werkzeughalter-Rohr (3), das koaxial in einem Tragrohr (4) gelagert ist,
(b) eine erste Schnellkupplung mit einer Halterung (3a, 3b) für ein Werkzeug (1) im genannten Werkzeughalter-Rohr (3),
(c) eine zweite Schnellkupplung (4b, 4c, 2b, 2c) in Verbindung mit dem genannten Tragrohr (4) und dem genannten Gehäuse (2b), das die Antriebswelle (2a) umschliesst und
(d) Sperrmittel (3b, 2d, 2e) in Verbindung mit dem genannten Werkzeughalter-Rohr (3) und der Antriebswelle (2a), so dass die Blockierung der genannten ersten Schnellkupplung mit Hilfe der genannten zweiten Schnellkupplung bewirkt wird.

2. Einrichtung für die Schnellkupplung nach Anspruch 1, **dadurch gekennzeichnet, dass** die genannte erste Schnellkupplung eine Schnappbefestigung (3a, 3b, 1c) ergibt, welche das Einführen und Einspannen eines Werkzeugs im Werkzeughalter-Rohr (3) sowie die rasche Freigabe des Werkzeugs gestattet.

3. Einrichtung nach Anspruch 2, **dadurch gekennzeichnet, dass** die genannte erste Kupplung bewegliche Kugeln (3a) aufweist, die in Löchern im genannten Werkzeughalter-Rohr (3) vorgesehen und einer elastischen Hülse zugeordnet sind, die an diesem Werkzeughalter-Rohr (3) befestigt ist, so dass diese Kugeln in einer inneren Stellung festgehalten sind, in der sie in das Werkzeughalter-Rohr (3) eindringen, um das Werkzeug zu blockieren und dass diese Kugeln nach aussen gedrückt werden können, um das Werkzeug freizugeben.

4. Einrichtung nach Anspruch 2 oder 3, **dadurch gekennzeichnet, dass** der Durchmesser des genannten Werkzeughalter-Rohrs (3) den auswechselbaren Werkzeugen angepasst ist, die einen runden Schaft mit einem Durchmesser aufweisen, der das Einführen mit einem geringen Spiel in das Werkzeughalter-Rohr (3) gestattet und mit einer der genannten Schnappbefestigung (3a, 3b, 1c) zugeordneten ringförmigen Nute (1c) sowie einem Kupplungsnocken (ld) versehen ist,.

5. Einrichtung nach einem der Ansprüche 1 bis 4, **dadurch gekennzeichnet, dass** die genannte zweite Schnellkupplung in Form einer Muffenverbindung vorgesehen ist, wobei die zu verbindenden Enden des genannten Gehäuses (2b) der Antriebswelle (2a) und des Tragrohrs (4) so ausgebildet sind, dass sie das entsprechende Einsteck- bzw. Aufnahmeelement bilden.

6. Einrichtung nach Anspruch 5, **dadurch gekennzeichnet, dass** das Ende des Tragrohrs (4), das mit dem genannten Gehäuse (2b) der Antriebswelle (2a) verbunden wird, in Form einer Hülse (4b) gestaltet ist, die das Aufnahmeelement bildet und dem Gehäuse (2b) zugeordnet ist, welches das Einsteckelement der genannten Muffenverbindung bildet.

7. Chirurgisches Instrument mit einer Schnellkupplung nach einem der Ansprüche 1 bis 6, wobei auswechselbare Schneidewerkzeugen in verschiedenen vorbestimmten Abständen zu einer Antriebswelle befestigt werden, **dadurch gekennzeichnet, dass**
(a) das Instrument einen Satz auswechselbarer Zwischenspindeln in Verbindung mit einem Satz auswechselbarer Werkzeuge mit identischen Schäften umfasst, wobei diese Spindeln jeweils eine Länge aufweisen, die einem vorbestimmten Abstand zwischen Werkzeug und Antriebswelle (2) entsprechen und aus einem drehenden Werkzeughalter-Rohr (3) bestehen, das in einem Tragrohr (4) eingebaut ist und die genannten auswechselbaren Werkzeuge (1) mit Schneideköpfen (1a) in verschiedenen Formen und mit identischen Schäften (1b) versehen sind, deren Durchmesser und Länge vorbestimmt sind,
(b) das genannte Werkzeughalter-Rohr (3) einen Innendurchmesser aufweist, der das Einführen des Schafts (1a) eines Werkzeugs (1) mit geringem Spiel gestattet, wobei eine erste Schnellkupplung dem Werkzeughalter-Rohr (3) zugeordnet ist, um das Befestigen eines Werkzeugs zu ermöglichen und Kupplungsmittel vorgesehen sind, um das Kuppeln des Werkzeugs mit der Antriebswelle (2a) mit Hilfe dieses Werkzeughalter-Rohrs (3) zu gestatten,
(c) dem genannten Tragrohr (4) eine zweite Schnellkupplung zugeordnet ist, um das Befestigen dieses Tragrohrs (4) am Gehäuse (2b) der Antriebswelle (2a) zu ermöglichen und
(d) den genannten ersten und zweiten Schnellkupplungen Sperrmittel zugeordnet sind, deren Wirkung so gekoppelt ist, dass die Blockierung der ersten Schnellkupplung mit Hilfe der zweiten Schnellkupplung sichergestellt wird, um damit das Einspannen des Werkzeugs sicherzustellen, wobei das Trennen der zweiten Schnellkupplung die Freigabe des Werkzeugs gestattetet.

8. Chirurgisches Instrument nach Anspruch 7, **dadurch gekennzeichnet, dass** die erste Schnellkupplung eine Schnappbefestigung (3a, 3b) aufweist, die das Einbringen und die Halterung eines Werkzeugs im Werkzeughalter-Rohr (3) sowie die rasche Freigabe des Werkzeugs gestattet.

9. Chirurgisches Instrument nach Anspruch 8, **dadurch gekennzeichnet, dass** die genannte erste Schnellkupplung bewegliche Kugeln (3a) aufweist, die in Löchern im genannten Werkzeughalter-Rohr (3) vorgesehen sind und einer elastischen Hülse (3) zugeordnet sind, die auf dem genannten Werkzeughalter-Rohr (3) befestigt und so vorgesehen ist, so dass diese Kugeln in einer inneren Stellung festgehalten sind, in der sie in das Werkzeughalter-Rohr (3) eindringen, um die Halterung des Werkzeugs zu gewährleisten und dass diese Kugeln nach aussen gedrückt werden können, um das Werkzeug freizugeben.

10. Chirurgisches Instrument nach einem der Ansprüche 8 oder 9, **dadurch gekennzeichnet, dass** es einem Satz auswechselbarer Werkzeuge mit identischen runden Schäften (1b) umfasst, deren Durchmesser den Einbau im Werkzeughalter-Rohr (3) mit geringem Spiel gestattet und eine der Schnappbefestigung zugeordnete ringförmige Rille (1c) sowie eine Kupplungsnocken (ld) aufweisen.

11. Verfahren für die Schnellkupplung von auswechselbaren drehenden Werkzeugen mit einer Einrichtung nach Anspruch 1, **dadurch gekennzeichnet**:
(a) dass man Standardwerkzeuge mit Schneideköpfen jeder geeigneten Form verwendet, die auf identischen Schäften einer vorbestimmten Länge und eines vorbestimmtem Durchmessers befestigt sind, wobei dieser Durchmesser dem genannten drehbaren Werkzeughalter-Rohr angepasst ist, so dass der Schaft jedes Werkzeugs mit geringem Spiel in diesem Werkzeughalter-Rohr verschiebbar ist,
(b) dass man die Länge des Werkzeughalter-Rohrs dem erforderlichen Abstand zwischen der Antriebswelle und dem Schneidkopf jedem auf diesem Werkzeughalter-Rohr montierten Werkzeug anpasst, wobei dieses Werkzeughalter-Rohr im genannten drehenden Tragrohr so gelagert ist, dass es hierin drehbar ist,
(c) dass man eines der genannten Werkzeuge mit dem genannten Werkzeughalter-Rohr mit Hilfe der genannten ersten Schnellkupplung verbindet, so dass dessen axiale Stellung im genannten Werkzeughalter-Rohr festgelegt ist, und
(d) dass man das genannte Tragrohr anhand der genannten zweiten Schnellkupplung mit dem genannten Gehäuse der Antriebswelle verbindet, um die Kupplung der genannten Antriebswelle mit dem Schaft des im genannten Werkzeughalter-Rohr montierten Werkzeugs und zugleich das Verriegeln der genannten ersten und zweiten Kupplung derart zu bewirken, dass jedes so gekoppelte Werkzeug formschlüssig mit dem drehenden Werkzeughalter-Rohr und mit der Antriebswelle verbunden ist und dass zudem dieses Werkzeug freigegeben wird, sobald die zweite Schnellkupplung gelöst ist.

## Claims

1. Device for quick connection of interchangeable rotary tools to a drive shaft arranged in a housing connected to a removable support associated with means for coupling a tool with the drive shaft, **characterized in that** it comprises:
(a) a rotary tool-holder tube (3) mounted coaxially in a tubular support (4);
(b) a first quick connection comprising means (3a, 3b) for fastening a tool (1) in said tool-holder tube (3);
(c) a second quick connection (4b, 4c, 2b, 2c) associated with said tubular support (4) and with said housing (2b) disposed around the drive shaft (2a) and
(d) locking means (3b, 2d, 2e) associated with said tool-holder tube (3) and with the drive shaft (2a) in such manner as to cause locking of said first quick connection by means of said second quick connection.

2. Quick connection device according to claim 1, **characterized in that** said first quick connection comprises a snap coupling (3a, 3b, 1c) enabling a tool to be inserted and fixed in the tool-holder tube (3) as well as to be quickly released .

3. Device according to claim 2, **characterized in that** said first connection comprises movable balls (3a) accommodated in holes recessed in said tool-holder tube (3) and associated with an elastic sleeve (3b) fastened to said tool-holder tube (3) so that said balls are kept in an inner position wherein they penetrate into the tool-holder tube (3) in order to enable fastening the tool and that said balls may be driven outwards in order to liberate the tool.

4. Device according to claim 2 or 3, **characterized in that** the diameter of said tool-holder tube (3) is adapted to interchangeable tools comprising a round shank having a diameter enabling it to be mounted in the tool-holder tube (3) with a slight clearance, an annular groove (1c) associated with said snap coupling and a coupling projection (1c).

5. Device according to one of the claims 1 to 4, **characterized in that** said second quick connection device is provided in the form of a socket joint, the ends of said housing (2c) of the drive shaft (2a) and of the tubular support (4) to be connected being arranged so that they respectively constitute the male and female parts of said socket joint.

6. Device according to claim 5, **characterized in that** the end of said tubular support (4) to be connected to said housing (2b) of the drive shaft (2a) is provided with a socket (4b) constituting the female part and said housing (2b) is arranged so as to constitute the male part of said socket joint.

7. Surgical instrument provided with a quick connection according to one of the claims 1 to 6 in order to drive interchangeable cutting tools at different predetermined distances from a drive shaft, **characterized in that**:
(a) the instrument is provided with a set of interchangeable intermediate spindles associated with a set of interchangeable tools (1) provided with identical shanks, said spindles each having a length which corresponds to a predetermined distance between each tool and the drive shaft (2), each of said spindles consisting of a rotary tool-holder tube (3) mounted in a support tube (4) and said interchangeable tools (1) having cutting heads (1a) of different forms and identical shanks (1b) having a predetermined diameter and length,
(b) said tool-holder tube (3) has an inner diameter enabling the insertion of the shank (1a) of each tool (1) with a slight clearance, a first quick connection being associated with said tool-holder tube (3) so as to enable fastening each tool and coupling means being provided whereby to enable coupling the tool with the drive shaft (2a) by means of said tool-holder tube (3),
(c) said support tube (4) is associated with a second quick connection arranged to enable fastening said support tube (4) to said housing (2b) of the drive shaft (2a) and
(d) said first and second quick connections are associated with locking means having a combined action so that locking said first quick connection is ensured by means of said second quick connection in order to thereby ensure fastening of the tool, while the separation of said second quick connection enables the release of the tool.

8. Surgical instrument according to claim 7, **characterized in that** said first quick connection comprises a snap coupling (3a, 3b) which enables the insertion and fastening of a tool in the tool-holder tube (3) as well as quick release of the tool.

9. Surgical instrument according to claim 8, **characterized in that** said first connection comprises movable balls (3a) accommodated in holes recessed in said tool-holder tube (3) and associated with an elastic sleeve (3b) fastened to said tool-holder tube (3) and arranged so as to keep said balls in an inner position wherein they penetrate into the tool-holder tube (3) in order to enable fastening the tool and so that said balls may be driven outwards to release the tool.

10. Surgical instrument according to one of claims 8 or 9, **characterized in that** it is provided with a set of interchangeable tools comprising identical round shanks (1b) which have a diameter enabling it to be mounted with a slight clearance in said tool-holder tube (3), an annular groove (1c) associated with said snap coupling and a coupling projection (1d).

11. Method of quick connection of interchangeable rotary tools by means of a device according to claim 1, **characterized by**:
(a) using standard tools comprising cutting heads of any appropriate shape fastened to identical round shanks having a predetermined length and diameter, this diameter being adapted to said rotary tool-holder tube so as to enable the shank of each tool to slide with a slight clearance in this tool-holder tube
(b) adapting the length of said tool-holder tube to the required distance between the drive shaft and the cutting head of each tool mounted in said tool-holder tube, said rotary tool-holder tube being mounted in said tubular support so that it can rotate in said support.
(c) connecting each of said tools to said tool-holder tube by means of said first quick connections so as to fix its axial position in said tool-holder tube and
(d) connecting said tubular support to said housing of the drive shaft by means of said second quick connection, so as to effect coupling of said drive shaft with the shaft of the tool mounted in said tool-holder tube and to at the same time effect locking of said second connection and said first connection, in such manner that each tool thus connected is made completely solid with said rotary tool-holder tube and with said drive shaft and to enable the release of said tool as soon as said second connection is disengaged.
